Europäisches Patentamt

(19) European Patent Office

Office européen des brevets

(11) Publication number: **0 158 609**
**A2**

# (12) EUROPEAN PATENT APPLICATION

(21) Application number: 85830067.6

(22) Date of filing: 18.03.85

(51) Int. Cl.⁴: **A 61 K 6/10**

(30) Priority: 27.03.84 IT 936384

(43) Date of publication of application:
16.10.85 Bulletin 85/42

(84) Designated Contracting States:
AT BE CH DE FR GB LI LU NL SE

(71) Applicant: Cozzi, Gualtiero
28 Via Domenico Buonvicini
I-50132 Firenze(IT)

(72) Inventor: Cozzi, Gualtiero
28 Via Domenico Buonvicini
I-50132 Firenze(IT)

(74) Representative: Mannucci, Gianfranco, Dott.-Ing.
Ufficio Tecnico Ing. A. Mannucci Via della Scala 4
I-50123 Firenze(IT)

(54) Procedure for taking and correcting dental impressions by means of alginate-based materials.

(57) When alginate-based materials are used, in order to obtain a sufficient adhesion between the material which constitutes the second impression and the material which constitutes the first impression, conditions are determined in the materials for the impressions, able to activate, with the retarder of the second impression material, the functional groups - blocked by the calcium - of the first impression material, so that bonds between the two materials of the two impressions are established.

**0158609**

<u>DESCRIPTION</u>

The technique of taking dental impressions alginate-based  is well known and has been applied for some decades. It is based on the preparation, upon the use, of a cream-like "mixture" obtained from a powder and water which sets rapidly, thus forming an elastic and tough mass. If the setting takes place after the "misture" has been introduced by means of a suitable "impression-holder" in the patient's mouth so as to envelope, for example, a whole dental arch, the impression of the latter remains faithfully imprinted in the mass.

It is known that the accuracy of the dental impression depends, among other things, on the fluidity and mouldability of the "mixture" put in contact with the teeth or tissues of the oral cavity. For this reason, the use of chromatic indicators for controlling the "working time" has remarkably contributed to better take even the sharpest details. In fact, the possibility of applying the "mixture" in the patient's mouth few seconds before the setting has started, allows to operate with "mixtures" much more fluid than those which- in the absence of chromatic indication - it would be necessary to apply in order to prevent the "mixture" from pouring down into the patient's throat.

The chromatic indication, though largely improving

- 1 -

the conditions for taking an impression to be used for the preparation of an accurate prothesis, does not assure a result with 100% of guarantee. An air bubble, for instance, unintentionally left in the point where the work has to be done, may make the impression obsolutely useless, and the whole operation must be repeated. Until now, working with alginated-based materials, any possible attempt to correct a faulty impression by the addition, at the considered point, of a small quantity of a new, fluid "mixture" and successive application in the patient's mouth, has not led to practical results. The added "mixture" does not show any adhesion to the underlying, already hardened material; its disjunction easily takes place upon withdrawing from the patient's mouth.

Therefore, this so-called dual impression technique, which is widely used to take dental impressions by means of silicones, has not, till now, been applied to alginates.

The following procedure makes possible the application of this dual impression technique even to the correction or to the improvement of the accuracy of an alginate-based impression, since it allows a good adhesion between the material of the first impression and the material of the second impression.

Accordingly, the object of this invention is a

- 2 -

0158609

procedure for improving or correcting flaws in dental impressions taken with alginate-based materials, by taking a second impression while using the first one as a support, according to which procedure - in order to achieve a sufficient adhesion of the second impression material to the first impression material - conditions are determined, at least in the first impression material, suitable to activate, with the retarder of the second impression material, the functional groups - blocked by the calcium - of the first impression material, so that chemical bonds between the materials of the two impress ions are established. Also in the second impression material conditions may be provided able to consent the activation of the functional groups in order to make up the bonds between the materials of the two impressions.

In practice, an excess of calcium salt may be avoided and, in particular, the quantity of calcium salt may be stoichiometrically limited  respect to the alginate present in the first impression.

The activity of the calcium ion may be limited indipendently from its concentration, by increasing the ionic strength present in the mixture prepared for taking the first impression. The calcium ion concentrat ion may be also limited by controlling the concentration.

of the anion which constitutes the calcium salt used for

- 3 -

the first impression.

Advantageously, the pH of the first impression may be buffered in such a way to maintain at a high value the concentration of the retarder which, being present in the fluid mass of the second impression, must go into the first one by diffusion. Magnesium dioxide may be advantageously used as a pH buffer agent in the material of the first impression.

Trisodium phosphate may be used as a retarder for the second impression.

A pH chromatic indicator may be introduced in the material of the second impression in order to detect the time useful for the application of the mixture for the second impression on the first one.

It is a further object of the Invention to provide an alginate-based material to perform dual impression operations, with the above mentioned criteria.

In order to better explain the grounds of the procedure, some general considerations on the alginates operating mechanism will be stated beforehand.

The powder consists of a soluble alginate and a calcium salt as main reagents, being necessary for the formation of the hardened mass which actually consists of calcium alginate. A retarding agent is present, consisting of soluble organic or inorganic salts which

- 4 -

provide, together with the calcium, insoluble products or which carry out a complexing action over the calcium so that to hinder for some time its reaction with the alginate. This permits to carry out all the preparing operations which preceed the application of the material in the patient's mouth.

In addition to these basic components, several correctives are present, together with the alginates, to improve the quality of the material making up the impression and an inert support as well having the purpose to allow for an easy mixing of the powder with water and to give to the whole a suitable "skeleton".

To make possible the adhesion of the material constituting the second impression to the material of the first one, it is necessary that the retarder, which is present and active during the period of fluidity of the "mixture" prepared for the second impression which is put in contact with the first impression material, is able to "activate" the alginate functional groups – blocked by the calcium – of the first impression, so that they are rendered utilizable for constituting chemical bonds between the first and the second impression.

This can be done:

a) By using, as a retarder in the "mixture" forming the

0158609

second impression, a salt showing a complexing or precipitating action on the calcium with an effect much higher than the one which is strictly sufficient to hinder its reaction with the alginate.

b) By spreading the "mixture" for the second impression on the first one within the time the retarder concentration is high so that its utmost mass-action can be exploited. This time, measured starting from the beginning of the mixing operation is not very long, but may be stoichiometrically controlled or, better, if the trisodium phosphate retarder is used, may be controlled by using a pH chromatic indicator showing a change in colour when the pH of the mixture goes below 11-10,5.

c) By formulating the powder for the first impression so that a calcium salt excess respect to the alginate is avoided, since this excess would impede the above described procedure for the activation of the alginate functional groups.

This limitation may be carried out by either metering the powder components so that the calcium salt will result in a stoichiometric amount lower than the equivalent sum of alginate and retarder; or by limiting the activity of the calcium ion by controlling the ionic strength of the medium, or by acting upon the concentration of the anion making up the calcium salt.

- 6 -

d) By preventing, through the presence of a buffer agent, the pH of the gelled "mixture" constituting the first impression, from going below a minumum level compatible with a sufficient "activity" of the retarding agent present in the "mixture" of the second impression, which must go into the first one by diffusion.

e) Owing to the fluidity of the second impression mixture which would tend to easily pour, it is useful to introduce a chromatic indicator to limit at the utmost the permanence of the "mixture" in the mouth before the gelling starts. If the trisodium phosphate retarder is used a pH chromatic indicator may be employed showing colour changes ranging about 10-9,5 pH.

C L A I M S

1. A procedure to improve or correct flaws in dental impressions taken with alginated-based materials by taking a second impression, the first one being used as a support; characterized in that - to achieve a sufficient adhesion of the second impression material to the first impression material - conditions are determined at least in the first impression material, able to activate with the retarder of the second impression material the functional groups - blocked by the calcium - of the first impression material, so that bonds between the materials of the two impressions are made up.

2. A procedure according to claim 1, characterized in that also in the second impression material conditions are determined able to consent the activation of the functional groups to make up the chemical bonds between the materials of the two impressions.

3. A procedure according to claim 1, characterized in that an excess of calcium salt is avoided and in particular the quantity of calcium salt is stoichiometrically limited respect to the alginate present in the first impression.

4. A procedure according to claim 1, characterized in that the activity of the calcium ion is limited

- 1 -

0158609

indipendently of its concentration, by increasing the
ionic strength present in the mixture prepared for
achieving the first impression.

5. A procedure according to claim 1, characterized
in that the concentration of the calcium ion is limited
by controlling the concentration of the anion which
constitutes the calcium salt used for the first
impression.

6. A procedure according to claim 1, characterized
in that the first impression pH is buffered so as to
keep high the concentration of the retarder which,
being present in the fluid mass of the second impression,
must go by diffusion into the first one.

7. A procedure according to claim 6, characterized
by the use of magnesium oxide as a pH buffer agent in
the first impression material.

8. A procedure according to claim 2, characterized
by the use of trisodium phosphate - per se known -  as
a retarder for the second impression.

9. A procedure according to claim 2, characterized
by the use, in the second impression material, of a pH
chromatic indicator for measuring the time available
for the application of the mixture for the second
impression on the first one.

10. A procedure according to claims 1 and 2,

- 2 -

characterized by the use, as a retarder in the mixture for the second impression, of a salt showing a complexing or precipitating action over the calcium, which is higher than the one being strictly sufficient to prevent its reaction with the alginate, being the "mixture" for the second impression applied on the first one within the time during which the concentration of the retarder in said material for the second impression is still high, so that its utmost mass-action can be exploited.

11. A procedure according to claim 10, characterized by the use - to measure said time starting from the beginning of the mixing operation - of a pH chromatic indicator which shows colour change when the pH of the mixture for the second impression goes below 11-10,5, by using trisodium phosphate as a retarder.

12. A procedure as herein defined and for the specified purposes.

13. An alginate-based material for dental impressions, to perform dual impression operations, as described.